# EUROPEAN PATENT SPECIFICATION

(11) **EP 0 675 190 B1**
(45) Date of publication and mention of the grant of the patent: **21.09.2011**
(21) Application number: 95104231.6
(22) Date of filing: 22.03.1995
(51) Int. Cl.: C10G 70/04, F25J 3/02

(54) **Olefin recovery method**
Verfahren zur Rückgewinnung von Olefinen
Procédé pour la récupération d'oléfines

(30) Priority: 01.04.1994 US 221908
(43) Date of publication of application: 04.10.1995
(73) Proprietor: Kellogg Brown & Root LLC, Houston, TX 77210-4557 (US)
(72) Inventor: Verma, Vijender Kumar, Sugar Land, Texas 77478 (US)
(74) Representative: Dey, Michael

(56) References cited:
- US-A- 2 933 901
- US-A- 3 902 329
- US-A- 4 163 652
- US-A- 4 601 739

## Description

### Field of the Invention

The present invention relates to enhanced olefins recovery in an olefins plant wherein a liquid hydrocarbon conditioning stream is injected into a cracking furnace effluent stream to reduce refrigeration energy consumption.

### Background of the Invention

US 4,601,739 describes a process for fractional distillation. US 4,601,739 concerns the improvement of the operation of a demethanizer. Thereby, auxiliary material is added to the reflux stream to increase the reflux volume.

US 2,933,901 describes separation of cracking effluents using a fractionation column. According to US 2,933,901 the fractionation column is operated as reboiled absorber, whereby a solvent such as liquid propylene is introduced into the upper region of the column to absorb constituents heavier than methane. Thus, the liquid solvent is added as a reflux solvent.

US 4,163,652 describes a method for refrigerative fractionation of cracking gases. US 4,163,652 thereby teaches to use ethane as refrigeration fluid, since very low temperatures down to -88 °C are obtainable therewith.

US 3,902,329 describes distillation of methane and hydrogen from ethylene. In the procedure described in US 3,902,329 an additional reflux stream is introduced at the top of a fractionation column.

Ethylene is a ubiquitous building block in the manufacture of a wide variety of chemical and plastic products. Ethylene is typically produced industrially by pyrolysis of hydrocarbons in a furnace in the presence of steam. The furnace effluent stream comprising a range of components is typically cleaned up, dried to remove water, compressed and passed to an olefins recovery section to condense the ethylene and other condensable heavy end components (ethane, propylene, propane, etc.). The condensed stream is then distilled to remove the light ends (methane and hydrogen) and fractionated to separate ethylene from the heavy ends.

Compositional range of the furnace effluent stream depends on several factors including the type of hydrocarbon feedstock used. A representative composition of the effluent of a furnace employing three different hydrocarbon feedstocks and operated to maximize ethylene formation is given in Table 1.

**Table 1**

| Component | Effluent Composition (mole %) | | |
|---|---|---|---|
| | Furnace Feedstock | | |
| | Ethane | Propane | Naphtha |
| H₂ | 35.9 | 20.5 | 15.8 |
| CH₄ | 6.5 | 27.8 | 26.5 |
| C₂H₄ | 34.3 | 32.0 | 26.5 |
| C₂H₆+ | 23.3 | 19.7 | 24.1 |

As can be seen, hydrogen and methane light end components comprise a substantial portion of the effluent. These light ends have an undesirable impact on the stream dew point temperature. Greater refrigeration power is required to condense out ethylene and other components from streams containing high hydrogen and methane concentration, and refrigeration makes up a significant portion of the process energy requirements. Additionally, in existing plants ethylene refrigeration availability may be limited and therefore a process bottleneck to any increase in ethylene output.

It would be desirable to compensate for the presence of light end components to obtain greater condensation against propylene refrigeration. As far as applicant is aware, in an ethylene plant employing hydrocarbon pyrolysis, it has been heretofore unknown to reinject liquid hydrocarbons, particularly C₂-lean liquid hydrocarbons from the liquid driers, deethanizer and/or depropanizer into the reactor effluent stream for the purpose of raising the stream dew point temperature, lowering refrigeration energy usage and shifting cooling requirements from ethylene refrigeration to propylene refrigeration.

### Summary of the Invention

The invention relates to a method for recovering olefins according to claim 1.

Injection of liquid hydrocarbons into the reactor effluent stream prior to the bulk of the refrigeration imput in an olefins plant can raise the dew point temperature of condensing streams and shift refrigeration requirements from relatively colder ethylene refrigeration to relatively warmer propylene refrigeration to reduce energy usage. The injected liquid can comprise drier liquids condensed from the furnace effluent following the compression area, condensate recovered from the chilling train, C₂-lean products recycled from the deethanizer and/or depropanizer distillation columns or combinations thereof. In addition, the liquid hydrocarbon can be from an outside source such as propane and/or propylene introduced into the process.

The present invention provides a method for recovering olefins from a stream of light hydrocarbons containing hydrogen and methane, which is a suitably treated cracking furnace effluent stream. In one step, a liquid hydrocarbon conditioning stream is injected into the furnace effluent stream to form a conditioned stream. In another step, olefins from the conditioned stream are condensed and recovered through a series of chilling and vapor-liquid separation steps. The condensed olefins are further treated in a methane separator to separate methane and hydrogen. The liquid hydrocarbon conditioning stream comprises a drier liquid stream, C₂-lean deethanizer bottoms stream, C₂-lean depropanizer overhead or bottoms stream, or a combination thereof. Preferably, olefins and heavier components in the conditioned stream are partially condensed and recovered in a propylene refrigerant primary chilling and vapor-liquid separation step to form a primary lean vapor stream and a primary olefins condensate stream. Olefins from the primary lean vapor stream are then further condensed in successive chilling and separation steps.

The methane separator comprises a demethanizer distillation column. The primary condensed olefins stream can be stripped of methane and lighter components in a prestripper or fed to the demethanizer distillation step.

The present method also can be practiced in a depropanizer-first arrangement. In the depropanizer-first arrangement, C₄₊ components are substantially separated from the treated furnace effluent stream prior to the liquid injection step. In the depropanizer-first arrangement, the primary olefins condensate stream is preferably stripped in the prestripper to produce an enriched condensate stream and a secondary lean vapor stream. The enriched condensate stream is then fed to a deethanizer. The secondary lean vapor stream is fed to the methane separator.

An olefins plant comprises a furnace unit for cracking hydrocarbons and producing an effluent stream comprising hydrogen and olefins. The plant includes a line for injecting a liquid hydrocarbon conditioning stream into the effluent stream and producing a conditioned stream having a lower vapor content at primary refrigerant temperature. A series of cascaded condensers and vapor liquid separators are adapted to condense and recover olefins from the conditioned stream. The plant also includes a methane separator such as a demethanizer distillation column for removing methane from the condensed olefins, and a refrigeration system for supplying the primary refrigerant to one or more of the cascaded condensers. The olefins plant preferably includes a unit for treating the furnace effluent stream upstream from the liquid injection line. The treating unit includes a compressor and a drier in series, optionally with a cooler, a chiller, an acid gas removal unit or a combination thereof. The olefins plant preferably comprises a primary condenser operatively associated with a primary vapor-liquid separator for partially condensing olefins from the conditioned stream to produce a primary lean vapor stream for feed to the cascaded condensers and separators.

The olefins plant can use a deethanizer-first or a depropanizer-first scheme, that is, the plant can include a distillation unit for substantially separating C₂ or C₃, respectively, and heavier components from the treated furnace effluent before the liquid hydrocarbon conditioning line.

In another embodiment of the olefins plant, a series of cascaded cross-exchangers are preferably provided for partially condensing olefins from a portion of the furnace effluent stream by heat exchange against the cooled olefins-lean vapor and recovered hydrogen and methane streams. Preferably, an expander is provided to expand and further cool the olefins-lean vapor and lines are provided for directing the cooled olefins-lean vapor and recovered hydrogen and methane streams as heat exchange media to the cross-exchangers.

A further embodiment of the present invention provides an improvement to a method for recovering olefins from a cracking furnace effluent stream containing olefins. The method includes the steps of condensing and recovering olefins from the furnace effluent stream through a series of chilling and vapor-liquid separation steps, including partially condensing olefins and heavier components from the furnace effluent stream in a primary chiller and recovering condensed olefins and lean vapor in a primary vapor-liquid separator and condensing and recovering olefins from the lean vapor stream through a series of secondary chilling and vapor-liquid separation steps, and distilling the recovered olefins in a demethanizer. The improvement comprises the step of injecting a liquid hydrocarbon conditioning stream into the furnace effluent stream prior to the olefin condensation steps.

### Brief Description of the Drawings

Fig. 1 is a flow diagram of an ethylene plant of the present invention including injection of a liquid hydrocarbon conditioning stream to the chilling train, wherein the conditioning stream comprises a C₂-lean stream recycled from the deethanizer and/or the drier liquids from the compressor area and the plant has a demethanizer-first arrangement.
Fig. 2 is a schematic diagram of the chilling train of an ethylene plant of Fig. 1 including a membrane hydrogen separator unit.

### Detailed Description of the Invention

In the present invention, liquid hydrocarbons condensed from the various process units (e. g. the compression unit, deethanizer, depropanizer, chilling train, etc.) can be reinjected at one or more locations upstream from a methane separation unit of a chilling train to effect stream conditioning and increase the amount liquids condensed against warmer temperature refrigerants such as propylene refrigeration.

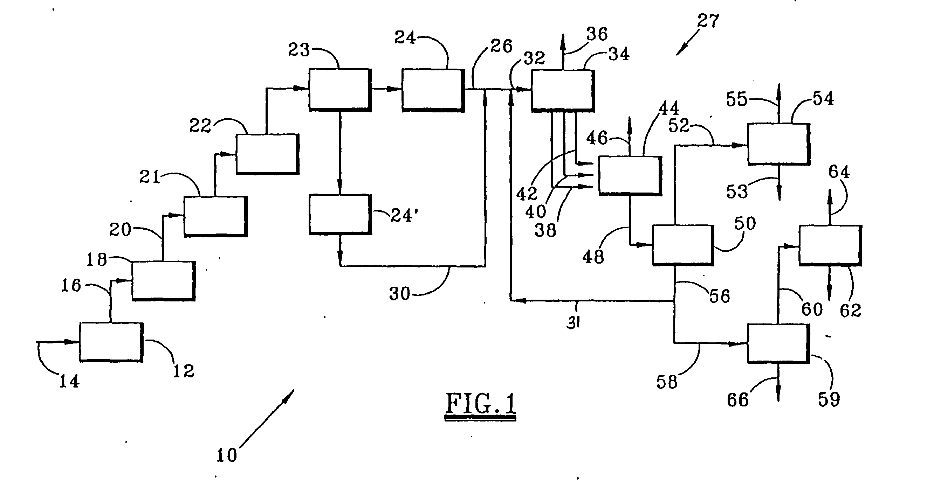

Referring to Figs. 1-2, wherein like referenced parts have like numerals, an olefins production plant 10 used in the method of the present invention comprises a cracking furnace **12** having reaction tubes (not shown) and a feed line **14** for the introduction of a hydrocarbon feedstock such as ethane, propane, butane, naphthas, gas oil, other petroleum fractions or combinations thereof. As is well known in the petrochemical arts, the hydrocarbon feedstock is conventionally cracked by pyrolysis in the presence of steam to produce a raw multicomponent effluent stream 16 comprising olefins such as ethylene, propylene, butadiene, and the like. The raw effluent stream **16** also contains hydrogen, steam, and a range of hydrocarbon reactants and byproducts including methane, ethane, propane, butane, etc. The raw effluent stream **16** has a composition and yield which are dependent on several factors including feedstock type, steam content, conversion rate, and furnace temperature, pressure, residence time, severity, etc.

Following production in the furnace **12,** the raw effluent stream **16** is cooled in a heat recovery zone 18 generally by steam generation and/or one or more quenches with water and/or hydrocarbon streams wherein process heat can be recovered for other uses. The raw, quenched effluent **20** can be optionally distilled in a primary fractionation cooling zone (not shown) to separate heavy fractions and to knock out steam condensate. Following any primary fractionation and/or cooling quenching steps, the vapors are compressed in first-compression zone 21 to a pressure suitable for an acid gas removal zone **22** for removing H₂S and CO₂, if necessary. The acid gas removal zone **22** generally comprises conventional scrubbers using agents such as caustic and/or amines. The desulfurized effluent is then compressed in a second compression zone **23** to a pressure suitable for subsequent cryogenic olefins recovery-- typically to a final pressure of from about 2.0 to about 5.0 MPa. As used herein, all pressures are denoted as absolute pressure unless gauge pressure is indicated. Following compression and acid gas removal, the gas is generally dried to remove residual water using a desiccant such as a molecular sieve, for example, in a drier **24** to prevent the formation of ice or hydrates during subsequent cooling. The dried furnace effluent vapor stream 26, thus treated for sulfur and water removal and compressed, is passed to an olefins recovery train **27.** In the olefins recovery train, as described hereinbelow, the treated furnace effluent stream **26** is typically separated into its various components including methane, ethane, ethylene, propane, propylene, butane, and the like.

In the practice of the present invention, the treated furnace effluent stream **26** is conditioned for enhanced olefins recovery against warmer temperature refrigerants such as propylene refrigeration by injection of a liquid hydrocarbon conditioning stream. The conditioning stream can comprise any liquid hydrocarbon process stream comprised mostly of C₃ and heavier components. One such available liquid hydrocarbon stream includes a drier liquid stream 30 comprising hydrocarbon liquids produced during compression and/or primary fractionation and dried in a drier **24'.** Additional liquid hydrocarbon conditioning streams are those C₂-lean streams which are recovered following processing for olefins separation.

As seen in Fig. 1, the drier liquid stream **30,** and/or a liquid hydrocarbon stream **31** recycled from the downstream olefins recovery train **27** are reinjected into the treated effluent stream **26** to form conditioned stream **32** for feed to the chilling train **34.** The overall amount of liquid hydrocarbon stream(s) recycled and reinjected should be sufficient to enhance liquid dropout against warmer refrigeration, but not so great as to excessively increase the size of the downstream separation equipment and associated heat exchangers.

The conditioned stream **32** is chilled in a cascaded chilling train **34** against refrigerants such as other process streams and/or propylene and ethylene using a cascaded series of condensation stages. The condensate is then separated from the vapor in a respective knock-out drum and the remaining gas is sent on for further treatment, e.g. further condensation, refrigeration recovery and/or hydrogen recovery. Three or more cascaded cooling stages are typically used. Cooling in the condensation stages is generally (but not necessarily) divided between a process cross-exchanger for an exchange of heat against one or more cold process streams and a refrigeration condenser for an exchange of heat against a refrigerant. The proportion of the split between the process cross-exchanger and the refrigeration condenser will depend on the amount of cooling available from the cold process stream(s).

A light end hydrogen-rich vapor stream 36 recovered from the chilling train **34** is typically used as a fuel or for hydrogen recovery. Several C₂₊ condensate streams **38, 40, 42** formed at the various cascaded stages of the chilling train **34** are fed to a methane separation unit **44** which is a demethanizer distillation column to separate residual light ends components (methane and hydrogen). A methane and residual hydrogen stream **46** is taken off overhead and can also be used for a fuel. A C₂₊ bottoms stream **48** is fed to a deethanizer **50.** In the deethanizer **50**, C₂'s are separated from C₃₊ components. A C₂'s stream **52** is fed overhead to an ethylene-ethane splitter **54** for fractionation in to an ethylene product stream **55** overhead and an ethane stream 53 recovered as a bottoms product.

A portion of the deethanizer bottoms stream **56** comprising C₃₊ components or another C₂-lean deethanizer side stream (not shown) is recycled as the liquid hydrocarbon conditioning stream **31** for reinjection into the treated furnace effluent stream **26.** Deethanizer bottoms not recycled can be fed through line **58 to** a depropanizer **59.** In the depropanizer **59,** C₃'s are separated from C₄₊ components. The C₃'s stream 60 can be fed to a propylene-propane splitter **62** for splitting a propylene product stream **64** overhead. The C₄₊ component bottoms stream 66 removed from the depropanizer **59** can be fed for further fractionation and recovery of heavier components as well known in the art.

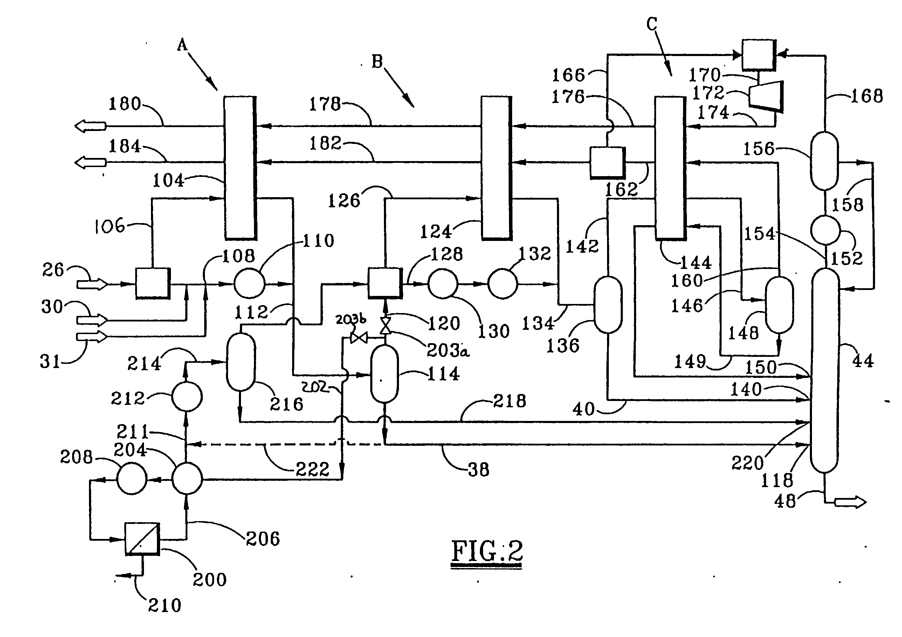

Further details of the conditioning steps, and the operation of the chilling train **34** and the methane separation unit **44,** are shown in Fig. 2. The chilling train **34** comprises a primary chilling and vapor-liquid separation stage **A** using process streams and/or liquid propylene as the primary refrigerant. Vapor not condensed stage **A** is passed to additional cascaded chilling and separation stages **B and C** wherein ethylene is the primary refrigerant.

The first separation stage **A** can include one or more cross-exchanger precoolers (not shown) comprising the reboilers of one or more downstream fractionation columns (such as the ethylene-ethane splitter). A portion of the gas stream **26,** generally minor, is directed through line **106** to a cross-exchanger **104** and the remaining portion is injected with the liquid hydrocarbon streams 30, 31 to form a conditioned stream **108** for enhanced olefins condensation against propylene refrigeration. The conditioned stream **108** is then directed to a first condenser **110** for partially condensing condensable olefin components therefrom. The cooled streams from the condenser **110** and cross-exchanger **104** are recombined and directed through line **112** to a first vapor-liquid separator drum **114.**

Chilling and conditioning of the mixed-phase feed to the first vapor-liquid separator drum **114** can generally occur in any order, including refrigeration in line **26** prior to or after the injection from lines 30 and 31. Where the conditioned stream **112** is at the lowest temperature feasible (usually -37 to -40°C) with a low-level refrigerant, e.g. propylene refrigerant, and contains the highest economic level of liquid reinjection, the quality of uncondensed vapor from the separator drum **114** will be at a minimum and will require less high-level refrigerant, e.g. ethylene refrigerant.

Olefins condensate from the bottom of the first drum **114 is** fed through line 38 to a relatively lower feed point 118 on the demethanizer **44.** Lean vapor from the drum **114** is directed through line 120 to a second condensation stage B wherein ethylene refrigerant is used. Similar to the first stage A, the vapor is divided with a portion directed to a second cross-exchanger cooler **124** through line **126** with the remaining portion passed though line **128** to condensers 130 and 132. The partially condensed split streams thus cooled are recombined and passed through line **134** to a second vapor-liquid separator drum **136.** Separated condensate from the second drum 136 is directed through line **40** to the demethanizer **44** at an intermediate feed point 140.

The condenser **130** typically operates at a temperature on the order of -60°C corresponding to the ethylene refrigerant at about -63°C. The condenser 132 typically operates at a temperature on the order of -83°C corresponding to the ethylene refrigerant at about -86°C. The pressure of the second condensation stage **B** is preferably similar to the pressure of the first condensation stage **A** (2.0 to 5.0 MPa).

Vapor from the second drum 136 is introduced through line **142** to a final condensation stage C wherein the primary refrigerant is preferably the lowest level of ethylene refrigerant and/or one or more cold process gas streams. From the drum **136,** the vapor is directed in full to a third cross-exchanger **144** wherein most of the methane and essentially all of the C₂ and heavier remaining condensable components are condensed by an exchange of heat with ethylene refrigerant and chilled process gas streams, e.g. light ends which are not condensed in the olefins recovery process. A partially condensed chilled stream **146** from the cross-exchanger **144** is passed to a third vapor-liquid separator drum **148.** A condensate stream **149** separated in the third drum **148** is first preferably passed through the cross-exchanger 144 as a cooling liquid. A partially heated third condensate stream is then fed via line **42** to the demethanizer **44** at a relatively higher feed point **150.**

The demethanizer **44** as known in the art can be a distillation column containing conventional internal vapor/liquid contacting devices such as, for example, packing shapes or trays. Overall dimensions and number of trays are specified by standard design criteria which in turn depend on composition of the several condensate feeds. The demethanizer shown in Fig. 2 operates substantially at the same pressure as the cascaded condensers so that the reflux liquid can be provided by an overhead partial condenser **152** using ethylene refrigerant. Alternatively, the demethanizer can be operated at a lower pressure using methane refrigerant. Overhead vapor from the demethanizer **44** is passed through line **154** to the condenser **152** wherein ethylene refrigerant is preferably used to condense condensable components. A partially condensed demethanizer overhead is passed to a condensate knock-out drum **156**. Condensate recovered from the overhead stream is recycled as reflux liquid to the demethanizer **44** through line 158. Cold overhead vapor components comprising light ends (mostly methane) separated from the olefin and heavy component liquids are directed, either with or without expansion in expansion stage **172**, to the cross-exchange coolers **144, 124,** and/or **104** as a cooling medium for recapture of a portion of the cooling energy. Note that any pressure can be selected for the operation of the demethanizer **44,** and various other methods of providing reflux can be used in the present invention. Also, any excess reflux provided by the overhead partial condenser 152 can be used as refrigerant in exchanger **144.**

The bulk of the demethanizer vaporization heat for vapor reflux is provided by a reboiler (not shown). The demethanizer reboiler can use a conventional low temperature heating medium such as propylene refrigerant to recover refrigeration.

Bottoms liquid comprising olefins and heavy ends from the demethanizer **44** is directed through line **48** for fractionation into individual components in a conventional refining zone such as shown in Fig. 1 comprising the deethanizer **50,** C₂ splitter **54,** depropanizer **59,** etc. as mentioned above.

Cold noncondensable vapor from the third drum **148** typically comprises hydrogen at an initial temperature of about -135°C, and may be further processed to improve hydrogen purity, for example, in one or more cascaded cooling zones (not shown). With or without such additional processing, the vapor is preferably used in a cascaded fashion as cooling media in the cross-exchangers **104, 124, 144.** The vapor from the third drum **148** is passed through line **160** as a cooling medium in the cross-exchanger **144** and then through lines **162** and **164** as a cooling medium in the cross-exchanger **124.** However, a portion or all of the stream **162** can be diverted through line **166** and combined with the cold light ends gas stream in line **168,** comprising primarily methane with some hydrogen and carbon monoxide from the demethanizer **44.** The combined stream **170** can be further cooled by expansion to a pressure of about 0.5 MPa, for example, in a turbine expander **172** to increase cooling capacity of the stream and recover power from the expansion. The proportion of the stream 162 diverted into line **166** generally depends on the chilling process cooling balance in accordance with standard engineering concepts.

The expanded, cooled stream from the expander **172** is directed through line **174** to cross-exchanger **144** as an additional cooling medium, and then through line **176** to exchanger **124** and through line **178** to exchanger **104.** At least a portion of the expanded, cooled stream from the expander **172** also can be employed as an additional cooling medium for the overhead vapor from demethanizer **44** in an exchanger means (not shown) located downstream of condenser **152** and upstream of knock-out drum **156.** A methane-rich fuel gas stream is recovered in line **180.** The remaining hydrogen from line **162** is passed through line **164** preferably to the cross-exchanger **124** as a cooling medium and through line **182** to the cross-exchanger **104** to provide a hydrogen-rich product in line **184.**

The present olefin recovery process can include an optional membrane separator unit **200.** The membrane separator can reject a substantial portion of the hydrogen contained in the furnace effluent stream (see Table 1 for a representative composition). The membrane separator unit **200** is preferably installed early in the chilling process **34** prior to the bulk of the refrigeration input in order to raise the effluent stream dew point temperature as early as feasible. The membrane separator **200** can be installed at other locations in the present olefins recovery process, but a location following the first condensate separation drum **114** is preferred because partial pressure of hydrogen is higher and overall flow is lower since a large portion of C₂'s and heavier components have already been condensed and removed. Following hydrogen rejection, a hydrogen-lean stream produced can be further chilled against propylene refrigerant to drop out additional liquids before being passed to the subsequent cascaded refrigeration stages **B,** C.

As seen in Fig. 2, all or part of the hydrogen-rich vapor from the first drum **114** is passed from line **120** to the membrane separator **200** via line **202.** Valves 203a and **203b** control flow to the membrane separator **200.** Prior to the membrane separator **200,** however, the vapor from the drum **114** is generally heated to suitable membrane operating conditions. Vapor in line **202** is preferably heated initially in a cross-exchanger **204** by an exchange of heat first against a hydrogen-lean impermeate stream **206** and then in a heater **208** by an exchange of heat against a suitable heating medium such as, for example, steam or hot water.

The membrane separator can comprise any membrane system which is substantially permeable for hydrogen and substantially impermeable for ethylene and heavier hydrocarbons. The membrane should also have other suitable characteristics including compatibility with the process stream, structural strength to endure high transmembrane pressure differential, an adequate flux for given separation parameters, and the like. Membrane systems which may be suitable are available commercially from various manufacturers and under various tradenames, such as, for example, UOP, Hydranautics, Toray, Toyobo, DuPont, Permasep, Aschi, Eltech Systems, Occidental Chemicals, Oxytech Systems, Monsanto, Medal, Dow Chemical, W.R. Grace, Separex, Delta Engineering, Ube and the like. A hydrogen-rich permeate stream is obtained via line **210.** Gas which does not permeate the membrane separator exits through line **206.** Further information regarding the membrane hydrogen separation unit **200** is described in commonly assigned U. S. Ser. No. 222 , 205, "Olefin Recovery Method," filed of even date herewith by Verma et al., which is hereby incorporated herein by reference.

The hydrogen-lean stream from the exchanger **204** is directed through line **211** to a post-membrane condenser **212** for further cooling and liquid condensation. The post-membrane condenser **212** also preferably cools against -40°C or warmer propylene refrigerant and the resulting partially condensed stream preferably flows through line **214** into a post-membrane vapor-liquid separator drum 216. Condensate from the drum **216** generally has a lower bubble point temperature than condensate from the first stage drum 114, and is directed through line **218** to a lower intermediate feed point **220** of the demethanizer **44.** Vapor from the drum 216 is fed to the second condensate stage B through line **224.** Alternatively, drum **216** can be by-passed and the effluent from condenser **212** fed directly to the second condensation stage **B.**

The membrane separator can be located anywhere between the treating unit, i.e., the drier **24,** and the demethanizer column **44.** Where depropanizer-first and/or deethanizer-first schemes are used, the membrane separation unit 200 is preferably after the depropanizer and/or deethanizer.

In the practice of the present invention, an intermediate demethanizer condenser (not shown) can be used to enhance overall energy efficiency of the cryogenic distillation and extend energy savings realized by use of liquid hydrocarbon reinjection and/or membrane hydrogen separation. Use of the intermediate condenser adjacent the lowermost feed point **118** can improve the energy efficiency of the distillation column **44** by shifting condensation cooling duty from the overhead condenser **152** to the intermediate condenser operating at a higher temperature. Thus, a lower quality refrigerant can be used as the cooling medium for the intermediate condenser, reducing the cooling duty on the overhead condenser **152** which requires colder refrigerant.

As other optional features, the first stage condensate stream 38 can be fed from the first drum **114** to the demethanizer **44** via a demethanizer feed prestripper column (not shown). An overhead prestripper condenser (not shown) preferably operating at about -37°C using propylene refrigerant provides liquid reflux to the prestripper. An overhead olefins stream (not shown) leaving the prestripper is then fed to the demethanizer **44.** A bottoms stream (not shown) comprising the C₂+ heavy components is withdrawn from the prestripper (not shown) for further processing.

In place of a prestripper, the condensate feed to the demethanizer **44** from the first condensate separation drum **114** can be subcooled.

When the membrane separator is used, hydrocarbon liquid from the first drum **114** (or any of the other liquid hydrocarbon source) can be reinjected through line **222** into thy hydrogen-lean stream 211 prior to any additional chilling against the propylene refrigerant. In addition, the condensate from the membrane drum **216** can be prestripped and/or subcooled prior to feed to the demethanizer **44.**

The present invention can be further described by reference to the following examples.

### Example 1

Computer simulations were undertaken on the present chilling train **34** (including the demethanizer and deethanizer) using ethane, propane and naphtha as feedstock. Simulation parameters include reinjection of compressor area drier liquids and/or hydrogen rejection to determine a comparative degree of olefins knocked out against propylene refrigeration for each case and feedstock type. When the hydrogen rejection unit is employed, reinjection of first stage drum liquids is also considered. Standardized ethylene process flow diagrams are based on a demethanizer-first scheme conforming to Figs. 1 and 2 except that a four-stage chilling train is used including a separate -1000°C ethylene refrigeration condenser and drum between chilling stages B and C. Yields for the feedstocks involved are based on actual plant results. Standard simulation methods were employed.

Simulation parameters include a 680 million kg/yr (1.5 billion lb/yr) production rate and a tolerable ethylene loss rate in the hydrogen rejection stream **210** of about 0.5 percent. Pressure of the inlet stream **26** following the compression zone is about 4.2 MPa (600 psia). Approximate composition of the inlet stream for the three feedstocks is given above in Table 1. For an ethane feedstock, composition of the membrane inlet stream is given in Table 2.

**TABLE 2**

| Component | Conc. (mole Z) |
|---|---|
| H₂ | 52.97 |
| CO | 0.06 |
| C₁ | 8.04 |
| C₂ | 38.72 |
| C₃ | 0.17 |
| C₄+ | 0.04 |

A typical commercially available, hollow-fiber membrane is assumed. The membrane operating temperature is set slightly lower than the manufacturer's maximum recommended temperature. A minimum reject hydrogen pressure is set so that the rejected hydrogen could be supplied to an existing fuel header without compression.

The amount of liquids condensed against propylene in the first condensation stage for the three feedstocks are given in Table 3 for the three cases simulated. Compressor area drier liquids reinjection is assumed except for the ethane feedstock in which case a low quantity of condensed liquids may not justify the economics.

The amount of liquids dropout against propylene refrigerant in the first condensation stage A is increased for the propane and naphtha feedstocks by compressing the incoming stream and reinjecting the compressor area drier liquids.

For the ethane case, the membrane is the most significant factor in increasing liquids dropout against propylene. For propane and naphtha, the membrane has a relatively small effect on increasing the amount of liquids dropout against propylene refrigerant in the first condensation stage, due in part to the fact that reinjection of dried liquid from the compressor area is very effective in dropping the liquid in the first drum. However, the amount of liquids dropout against propylene for these two cases is significantly increased by reinjecting the first drum liquids.

**TABLE 3**

| Case | Liquids dropout 1st drum 114 | Liquids dropout membrane drum 216 | Increase in liquids dropout over base case | Increase in liquids dropout due to 1st drum 114 reinjection |
|---|---|---|---|---|
| | (kg/hr) | (kg/hr) | (kg/hr) | (kg/hr) |
| Ethane Feedstock* | | | | |
| Without membrane separator (base case) | 82,570 | -- | -- | -- |
| With membrane separator | 87,020 | 45,120 | 49,570 | -- |
| With membrane separator and 1st drum 114 reinjection | 87,020 | 136,580 | 54,010 | 4440 |

| Propane Feedstock | | | | |
|---|---|---|---|---|
| Without membrane separator (base case) | 127,820 | -- | -- | -- |
| With membrane separator | 131,090 | 5320 | 8590 | -- |
| With membrane separator and 1st drum 114 reinjection | 131,090 | 152,200 | 24,380 | 15,790 |

| Naphtha Feedstock | | | | |
|---|---|---|---|---|
| Without membrane separator (base case) | 156,220 | -- | -- | -- |
| With membrane separator | 159,390 | 2170 | 5340 | -- |
| With membrane separator and 1st drum 114 reinjection | 159,390 | 177,200 | 20,980 | 15,640 |

| | | | | |
|---|---|---|---|---|
| *-no drier liquid reinjection. | | | | |

### Examples 2-4

An olefin plant computer simulation similar to the simulation performed in Example 1 was undertaken to determine the refrigeration power savings of reinjecting a C₂-lean deethanizer bottoms liquid stream 31 into the treated furnace effluent stream **26** except that the base case of this study was for an ethylene plant with an 80/20 ethane/propane feedstock and the production rate was 450 million kg/yr. Additional base case simulation assumptions are a 3-drum demethanizer-first chilling train and drier liquid reinjection.

In the simulation examples, liquid from the deethanizer bottoms is recycled to the front end of the demethanizer-first chilling train at reinjection rates of 0 kg/hr (Example 2), 18,200 kg/hr (40,000 lb, Example 3) and 36,400 kg/hr (80,000 lb/hr, Example 4). The deethanizer bottoms liquid is pumped, chilled to 15°C, and then mixed with the drier liquids before reinjection. Cooling water and cold propylene vapor are used as chilling media.

Results in terms of power requirements are given in Table 4. Due to the extra liquid (from the deethanizer bottoms), more C₂'s are condensed by cooling prior to the first drum including both the C₂ splitter cross-exchanger reboilers (located at the front of the chilling train) and the propylene refrigeration condenser. Therefore liquid from the first drum (-37C) contains more C₂'s and C₂'s to be condensed against ethylene refrigeration are considerably reduced. This lowers required ethylene refrigeration duty and power. Ethylene refrigeration condensing duty against -40°C propylene refrigeration is also reduced.

The use of deethanizer bottoms liquid recycle results in the several changes in operating conditions.

**Table 4**

| Process Unit | Example 2 | Example 3 | Example 4 |
|---|---|---|---|
| | Deethanizer bottoms recycle rate (kg/hr) | | |
| | 0 | 18,200 | 36,400 |
| (Refrigeration level and | Heat Duties (MMKcal/hr) | | |
| type) | | | |
| Condenser (12°C, PR) | 1.37 | | 1.37 |
| Condenser 110 (-40°C, PR) | 29.38 | | 26.96 |
| Condenser 130 (-60°C, ER) | 2.16 | | 1.20 |
| Condenser 132 (-83°C, ER) | 1.84 | | 1.37 |
| Net C₂ Stripper Reboiler (-11/-18°C) | 10.06 | | 7.00 |
| Deethanizer Condenser | 2.73 | | 3.52 |
| Demethanizer Stripper reboilers total (27°C) | 2.35 | | 4.24 |
| Demethanizer Condenser 156 (-100°C, ER) | 0.77 | | 0.71 |
| Demethanizer Reboiler, (9°C) | 2.66 | | 1.95 |

| | Power (kW) | | |
|---|---|---|---|
| ER | 2011 | 1715 | 1506 |
| PR | 13523 | 13175 | 13078 |
| Total | 15534 | 14890 | 14584 |
| Difference | - | 644 | 950 |
| Recycle pump | - | 24 | 48 |
| Net Difference (savings) | - | 20 | 902 |

| | | | |
|---|---|---|---|
| PR = Propylene refrigeration ER = Ethylene refrigeration | | | |

Demethanizer stripper bottoms temperature increases from 16°C to 27°C. Consequently, not all of the reboil duty can be used for propylene refrigeration subcooling. The increased liquid rates can potentially increase the size of the demethanizer stripper.

Deethanizer condenser duty is increased from 2.91 to 3.52 MMkcal/hr. This is a 20 percent increase in the required reflux rate, however, the vapor flow increases only by 4 percent. The bottom deethanizer reboil duty is increased from 2.54 to 4.06 MMkcal/hr for a fixed side reboil duty of 4.0 MMkcal/hr. This may not be the optimum configuration for the deethanizer as more side reboil duty and/or more feed preheat may be possible. Also the preferred point from which the liquid recycle stream is taken may be changed from the bottoms to the side draw. the bottoms temperature does not change from 74°C so fouling should not increase.

Ethylene refrigeration compressor power is reduced by 25 percent. Propylene refrigeration compressor also is reduced. This scheme can be useful for debottlenecking wherein the availability of ethylene refrigeration is a process limiting factor. Due to the low temperature in the chilling train, fouling should not increase due to the liquid recycle stream. Recycle of a deethanizer liquid stream for conditioning the furnace effluent stream is an efficient way to enhance energy savings of an existing plant and/or reduce bottlenecking due to limited ethylene refrigeration availability.

The present olefins recovery process is illustrated by way of the foregoing description and examples.

## Claims

1. A method for recovering olefins from a cracking furnace effluent stream of light hydrocarbons comprising olefins, methane and hydrogen, comprising the steps of:
directing a portion of the cracking furnace effluent stream comprising olefins, methane and hydrogen to a first cross-exchanger (104);
injecting a liquid hydrocarbon stream comprising C₃ and heavier hydrocarbons into the remaining portion of the light hydrocarbon cracking furnace effluent stream comprising olefins, methane and hydrogen to form a conditioned stream (108), such that the stream dew point temperature is raised;
condensing and recovering olefins from the conditioned stream (108) through a series of chilling and vapor-liquid separation steps, said steps comprising
directing the conditioned stream (108) to a first condenser (110), wherein propylene refrigerant is used;
directing cooled streams from the first condenser (110) and the first cross-exchanger (104) in the chilling and vapor-liquid separation steps through a line (112) at a temperature of -37 °C to -40 °C at 2.0 to 5.0 MPa to a first vapor-liquid separator drum (114);
feeding olefins condensate from the bottom of the first vapor-liquid separator drum (114) through a line (38) to a lower feed point (118) relatively to an intermediate feed point (140) and a higher feed point (150) on a demethanizer (44);
directing lean vapor from the first vapor-liquid separator drum (114) through a line (120) to a second condensation stage wherein ethylene refrigerant is used;
dividing the vapor with a portion directed to a second cross-exchanger cooler (124) through a line (126) with the remaining portion passed though a line (128) to second and third condensers (130) operated at a temperature on the order of -60 °C at 2.0 to 5.0 MPa and (132) operated at a temperature on the order of -83 °C at 2.0 to 5.0 MPa;
recombining the partially condensed split streams and passing through a line (134) to a second vapor-liquid separator drum (136);
directing separated condensate from the second vapor-liquid separator drum (136) through a line (40) to the demethanizer (44) at the intermediate feed point (140); and
introducing vapor from the second vapor-liquid separator drum (136) through a line (142) to a third cross-exchanger (144), wherein most of the methane and
essentially all of the C₂ and heavier remaining condensable components are condensed by an exchange of heat with ethylene refrigerant and chilled process gas streams ;
passing a partially condensed chilled stream (146) from the third cross-exchanger (144) to a third vapor-liquid separator drum (148); and
feeding the partially heated condensate stream from the third vapor-liquid separator drum (148) via a line (42) to the demethanizer (44) at the higher feed point (150).

2. The method of claim 1, wherein the liquid hydrocarbon stream comprises a drier liquid stream.

3. The method of claim 1, wherein the liquid hydrocarbon stream is a deethanizer bottoms stream comprising C₃ and heavier hydrocarbons.

4. The method of claim 1, wherein the liquid hydrocarbon stream is a depropanizer stream comprising C₃ and heavier hydrocarbons.

5. The method of claim 1, further comprising the step of subcooling the primary olefins condensate stream from the bottom of the first vapor-liquid separator drum (114) for the methane separation step.

6. The method of claim 2, comprising partially condensing and recovering olefins from the conditioned stream in a primary chiller and vapor-liquid separator to produce a primary lean vapor stream and a primary olefins condensate stream, and condensing olefins from the primary lean vapor stream in successive chilling and separation steps.

7. The method of claim 6, wherein the methane separation step comprises distilling methane from the olefins in a demethanizer distillation column.

8. The method of claim 6, further comprising the step of stripping methane and lighter components from the primary olefins condensate stream for feed to the methane separation step.

9. The method of claim 6, further comprising the step of subcooling the primary olefins condensate stream for the methane separation step.

10. The method of claim 6, including substantially separating C₄+ components from the treated furnace effluent stream in a depropanizer distillation column prior to the liquid injection step.

11. The method of claim 2, including substantially separating C₃+ components from the treated furnace effluent stream in a deethanizer distillation column prior to the liquid injection step.

12. The method of claim 10, including the steps of:
stripping the primary olefins condensate stream in a prestripper column to separate light components therefrom and produce an enriched condensate stream and a secondary lean vapor stream;
feeding the secondary lean vapor to the methane separation step; and
feeding the enriched condensate stream to a deethanizer distillation column.

13. An olefins plant, comprising:
a furnace unit (12) for cracking hydrocarbons and producing an effluent stream comprising hydrogen and olefins;
a line (14) for injecting a liquid hydrocarbon stream into the effluent stream (26) thereby producing a conditioned stream (32);
a series of cascaded condensers and vapor-liquid separators (27, 34) for condensing and recovering olefins from the conditioned stream (32), and producing a cooled olefin-lean vapor stream;
a methane separator (44) for recovering a methane stream from the condensed olefins; and
a refrigeration system for supplying the primary refrigerant to one or more of the cascaded condensers comprising a unit for treating the furnace effluent stream upstream from the liquid injection line, including a compressor and a drier in series, further including:
a series of cascaded cross-exchangers (104, 124, 144) for partially condensing olefins from a portion of the furnace effluent stream by heat exchange against the cooled olefins-lean vapor and recovered methane streams;
an expander (172) for expanding and further cooling the olefins-lean vapor and recovered methane streams; and
lines (174, 176) for directing the cooled olefins-lean vapor and recovered methane streams as heat exchange media to the cross-exchangers (104, 124, 144).

14. The plant of claim 13, comprising a primary condenser operatively associated with a primary vapor-liquid separator for partially condensing olefins from the conditioned stream to produce a primary lean vapor stream for feed to the cascaded condensers and separators and a primary olefins condensate stream.

15. The plant of claim 14, wherein the methane separator (44) comprises a demethanizer distillation unit.

16. The plant of claim 14, comprising a depropanizer distillation unit (59) for substantially separating C₄ and heavier components from the treated furnace effluent before injection of the liquid hydrocarbon conditioning stream.

17. The plant of claim 16, including:
a prestripper for stripping the primary olefins condensate stream to substantially separate light end components therefrom and produce an enriched liquid stream and a secondary lean vapor stream;
a line for feeding the secondary lean vapor stream to the methane separation unit; and
a line for feeding the enriched liquid stream to a deethanizer distillation column.

## Patentansprüche

1. Verfahren zum Gewinnen von Olefinen aus dem Ausstrom eines Cracking-Ofens von leichten Kohlenwasserstoffen, umfassend Olefine, Methan und Wasserstoff, umfassend die Schritte:
Leiten eines Teils des Ausstroms des Cracking-Ofens, der Olefine, Methan und Wasserstoff enthält, zu einem ersten Kreuzaustauscher (104);
Einspritzen eines Dampfes flüssiger Kohlenwasserstoffe, umfassend C₃- und schwerere Kohlenwasserstoffe, in den restlichen Teil des Ausstroms leichter Kohlenwasserstoffe des Cracking-Ofens, der Olefine, Methan und Wasserstoff umfasst, um einen konditionierten Strom (108) zu bilden, so dass die Strom-Taupunkttemperatur erhöht wird;
Kondensieren und Gewinnen von Olefinen aus dem konditionierten Strom (108) durch eine Reihe von Kühl- und Dampf-Flüssig-Trennschritte bzw. Separationsschritte, wobei die Schritte umfassen:
Leiten des konditionierten Stroms (108) zu einem ersten Kühler (110), worin Propylen-Kühlmittel verwendet wird;
Leiten gekühlter Ströme von dem ersten Kühler (110) und dem ersten Kreuzaustauscher (104) in die Kühl- und Dampf-Flüssig-Separationsschritte durch eine Leitung (112) bei einer Temperatur von -37 °C bis -40 °C bei 2,0 bis 5,0 MPa zu einer ersten Dampf-Flüssig-Separatortrommel (114);
Einspeisen von Olefinkondensat vom Boden der ersten Dampf-Flüssig-Separatortrommel (114) durch eine Leitung (38) zu einem tieferen Einspeisungspunkt (118), relativ zu einem Zwischeneinspeisungspunkt (149), und einem höheren Einspeisungspunkt (150) auf einem Entmethanisierer (44);
Leiten von magerem Dampf von der ersten Dampf-Flüssig-Separatortrommel (114) durch eine Leitung (120) zu einer zweiten Kondensationsstufe, worin Ethylenkühlmittel verwendet wird;
Aufteilen des Dampfes, wobei ein Teil zu einem zweiten Kreuzaustauscherkühler (124) geleitet wird durch eine Leitung (126), wobei der restliche Teil durch eine Leitung (128) zu zweiten und dritten Kondensatoren (130) geleitet wird, betrieben bei einer Temperatur in der Größenordnung von -60 °C bei 2,0 bis 5,0 MPa und (132), betrieben bei Temperatur in der Größenordnung von -83 °C bei 2,0 bis 5,0 MPa;
Wiedervereinigen der teilweise kondensierten geteilten bzw. getrennten Ströme und Leiten durch eine Leitung (134) zu einer zweiten Dampf-Flüssig-Separatortrommel (136);
Leiten von separiertem Kondensat von der zweiten Dampf-Flüssig-Separatortrommel (136) durch eine Leitung (40) zu dem Entmethanisierer (44) an dem Zwischeneinspeisungspunkt (140); und
Einleiten von Dampf von der zweiten Dampf-Flüssig-Separatortrommel (136) durch eine Leitung (142) zu einem dritten Kreuzaustauscher (144), worin das Meiste des Methans und im Wesentlichen alle C₂ und schweren verbleibenden kondensierbaren Komponenten kondensiert werden durch Wärmeaustausch mit Ethylen-Kühlmittel und gekühlte Prozessgasströme;
Leiten eines teilweise kondensierten gekühlten Stroms (146) von dem dritten Kreuzaustauscher (144) zu einer dritten Dampf-Flüssig-Separatortrommel (148); und
Einspeisen des teilweise erhitzten Kondensatstroms von der dritten Dampf-Flüssig-Separatortrommel (148) über eine Leitung (42) zum Entmethanisierer (44) an dem höheren Einspeisungspunkt (150).

2. Verfahren nach Anspruch 1, worin der flüssige Kohlenwasserstoffstrom ein Trockner-Flüssig-System umfasst.

3. Verfahren nach Anspruch 1, worin der flüssige Kohlenwasserstoffstrom ein Entethanisierer-Bodenstrom ist, der C₃- und schwerere Kohlenwasserstoffe umfasst.

4. Verfahren nach Anspruch 1, worin der flüssige Kohlenwasserstoffstrom ein Entpropanisierer-Strom ist, der C₃- und schwerere Kohlenwasserstoffe umfasst.

5. Verfahren nach Anspruch 1, weiterhin umfassend den Schritt des Unterkühlens des primären Olefinkondensatstroms vom Boden der ersten Dampf-Flüssig-Separatortrommel (114) für den Methan-Separationsschritt.

6. Verfahren nach Anspruch 2, umfassen teilweises Kondensieren und Gewinnen von Olefinen von dem konditionierten Strom in einem primären Kühler und Dampf-Flüssig-Separator, um einen primären mageren Dampfstrom und einen primären Olefinkondensatstrom zu erzeugen, und Kondensieren von Olefinen von dem primären mageren Dampfstrom in aufeinanderfolgenden Kühl- und Separationsschritten.

7. Verfahren nach Anspruch 6, worin der Methan-Separationsschritt Abdestillieren von Methan von den Olefinen in einer Entmethanisierer-Destillationssäule umfasst.

8. Verfahren nach Anspruch 6, weiterhin umfassend den Schritt des Strippens von Methan und leichteren Komponenten von dem primären Olefinkondensatstrom zum Einspeisen in den Methan-Separationsschritt.

9. Verfahren nach Anspruch 6, weiterhin umfassend den Schritt des Unterkühlens des primären Olefonkondensatstroms für den Methan-Separationsschritt.

10. Verfahren nach Anspruch 6, umfassend wesentliches Separieren von C₄₊-Komponenten von dem behandelten Ofenausstrom in einer Entpropanisierer-Destillationssäule vor dem Flüssigkeit-Einspritzschritt.

11. Verfahren nach Anspruch 2, umfassend wesentliches Separieren von C₃₊-Komponenten von dem behandelten Ofenausstrom in einer Entethanisierer-Destillationssäule vor dem Flüssigkeit-Einspritzschritt.

12. Verfahren nach Anspruch 10, umfassend die Schritte:
Strippen des primären Olefinkondensatstroms in einer Vorsfrippersäule zum Separieren leichter Komponenten davon und zum Erzeugen eines angereicherten Kondensatstroms und eines sekundären mageren Dampfstroms;
Einspeisen des sekundären mageren Dampfes in den Methanseparationsschritt; und
Einspeisen des angereicherten Kondensatstroms in eine Entethanisiererdestillationssäule.

13. Olefinanlage, umfassend:
eine Ofeneinheit (12) zum Cracken von Kohlenwasserstoffen und zum Herstellen eines Ausstroms, der Wasserstoff und Olefine umfasst;
eine Leitung (14) zum Einspritzen eines flüssigen Kohlenwasserstoffstroms in den Ausstrom (26), wobei ein konditionierter Strom (32) erzeugt wird;
eine Reihe von Kaskadenkühlern und Dampf-Flüssig-Separatoren (27, 34) zum Kondensieren und Gewinnen von Olefinen aus dem konditionierten Strom (32) und Erzeugen eines gekühlten Olefin-armen Dampfstroms;
einen Methan-Separator (44) zum Gewinnen eines Methanstroms aus den kondensierten Olefinen; und
ein Kühlsystem zum Zuführen des ersten Kühlmittels zu einem oder mehreren der Kaskadenkühler, umfassend eine Einheit zum Behandeln des Ofenausstroms oberstromig von der Flüssigkeitseinspritzleitung, einschließlich eines Kompressors und eines Trockners in Reihe, weiterhin umfassend:
eine Reihe von Kaskadenkreuzaustauschern (104, 124, 144) zum teilweisen Kondensieren von Olefinen von einem Teil des Ofenausstroms durch Wärmeaustausch gegen den gekühlten Olefin-armen Dampf und gewonnenen Methanströmen; und
Leitungen (174, 176) zum Leiten des gekühlten Olefin-armen Dampfs und gewonnener Methanströme als Wärmeaustauschmedien zu den Kreuzaustauschern (104, 124, 144).

14. Anlage nach Anspruch 13, umfassend einen primären Kühler, der operativ verbunden ist mit einem primären Dampf-Flüssig-Separator zum teilweisen Kondensieren von Olefinen von dem konditionierten Strom zum Erzeugen eines primären mageren Dampfstroms zum Einspeisen in die Kaskadenkühler und Separatoren und eines primären Olefinkondensatstroms.

15. Anlage nach Anspruch 14, worin der Methanseparator (44) eine Entmethanisierer-Destillationseinheit umfasst.

16. Anlage nach Anspruch 14, umfassend eine Entpropanisierer-Destillationseinheit (59) zum Wesentlichen Separieren von C₄- und schwereren Komponenten von dem behandelten Ofenausstrom vor Einspritzenn des flüssigen Kohlenwasserstoffkonditionierungsstroms.

17. Anlage nach Anspruch 16, umfassend:
einen Vorstripper zum Strippen des primären Olefinkondensatstroms, um im Wesentlichen leichte Komponenten davon zu separieren und zum Herstellen eines angereicherten flüssigen Stroms und eines sekundären magern Dampfstroms;
eine Leitung zum Einspeisen des sekundären mageren Dampfstroms in die Methanseparationseinheit; und
eine Leitung zum Einspeisen des angereicherten flüssigen Stroms in eine Entethanisierer-Destillationssäule.

## Revendications

1. Procédé de récupération d'oléfines à partir d'un flux d'effluents de four de craquage d'hydrocarbures légers comprenant des oléfines, du méthane et de l'hydrogène, comprenant les étapes consistant à :
diriger une partie du flux d'effluents de four de craquage comprenant des oléfines, du méthane et de l'hydrogène vers un premier échangeur croisé (104) ;
injecter un flux d'hydrocarbures liquides comprenant des hydrocarbures C₃ et plus lourds dans la partie restante du flux d'effluents de four de craquage d'hydrocarbures légers comprenant des oléfines, du méthane et de l'hydrogène pour former un flux conditionné (108), de telle sorte que la température du point de rosée du flux augmente ;
condenser et récupérer les oléfines à partir du flux conditionné (108) grâce à une série d'étapes de refroidissement et de séparation liquide-vapeur, lesdites étapes consistant à
diriger le flux conditionné (108) vers un premier condensateur (110), dans lequel un réfrigérant propylène est utilisé ;
diriger les flux réfrigérés depuis le premier condensateur (110) et le premier échangeur croisé (104) dans les étapes de refroidissement et de séparation liquide-vapeur à travers une conduite (112) à une température de -37°C à -40°C entre 2,0 et 5,0 MPa vers un tambour de séparateur liquide-vapeur (114) ;
amener un condensat d'oléfines depuis la partie inférieure du premier tambour de séparateur liquide-vapeur (114) à travers une conduite (38) vers un point d'amenée inférieur (118) relativement à un point d'amenée intermédiaire (140) et un point d'amenée supérieur (150) sur un déméthaniseur (44) ;
diriger la vapeur pauvre depuis le premier tambour de séparateur liquide-vapeur (114) à travers une conduite (120) vers un deuxième étage de condensation dans lequel du réfrigérant éthylène est utilisé ;
diviser la vapeur, une partie étant dirigée vers un deuxième réfrigérant d'échangeur croisé (124) à travers une conduite ((126), la partie restante traversant une conduite (128) vers les deuxième et troisième condensateurs (130) fonctionnant à une température de l'ordre de -60°C entre 2,0 à 5,0 MPa et (132) fonctionnant à une température de l'ordre de -83°C entre 2,0 et 5,0 MPa ;
recombiner les flux séparés condensés partiellement et les faire passer à travers une conduite (134) vers un deuxième tambour de séparateur liquide-vapeur (136) ; diriger le condensat séparé depuis le deuxième tambour de séparateur liquide-vapeur (136) à travers une conduite (40) vers le déméthaniseur (44) au niveau du point d'amenée intermédiaire (140) ; et
introduire de la vapeur depuis le deuxième tambour de séparateur liquide-vapeur (136) à travers une conduite (142) vers un troisième échangeur croisé (144), dans lequel la plus grosse partie du méthane et essentiellement tous les composants condensables résiduels de C₂ et plus lourds sont condensés grâce à un échange de chaleur avec les flux de réfrigérant éthylène et de gaz de transformation réfrigérés ; faire passer un flux réfrigéré partiellement condensé (146) du troisième échangeur croisé (144) vers un troisième tambour de séparateur liquide-vapeur (148) ; et amener le flux de condensat partiellement chauffé du troisième tambour de séparateur liquide-vapeur (148) par le biais d'une conduite (42) vers le déméthaniseur (44) au niveau du point d'amenée supérieur (150).

2. Procédé selon la revendication 1, dans lequel le flux d'hydrocarbures liquides comprend un flux de siccatif liquide.

3. Procédé selon la revendication 1, dans lequel le flux d'hydrocarbures liquides est un flux de fond de dééthaniseur comprenant des hydrocarbures C₃ et plus lourds.

4. Procédé selon la revendication 1, dans lequel le flux d'hydrocarbures liquides est un flux de dépropaniseur comprenant des hydrocarbures C₃ et plus lourds.

5. Procédé selon la revendication 1, comprenant en outre l'étape consistant à sous-refroidir le flux de condensat d'oléfines primaire depuis la partie inférieure du premier tambour de séparateur liquide-vapeur (114) pour l'étape de séparation de méthane.

6. Procédé selon la revendication 2, consistant à condenser et récupérer partiellement les oléfines issues du flux conditionné dans un refroidisseur primaire et un séparateur liquide-vapeur pour produire un flux de vapeur pauvre primaire et un flux de condensat d'oléfines primaires, et à condenser les oléfines issues du flux de vapeur pauvre primaire dans des étapes successives de refroidissement et de séparation.

7. Procédé selon la revendication 6, dans lequel l'étape de séparation du méthane consiste à distiller le méthane à partir des oléfines dans une colonne de distillation de déméthaniseur.

8. Procédé selon la revendication 6, comprenant en outre l'étape consistant à réaliser le strippage du méthane et de composants plus légers à partir du flux de condensat d'oléfines primaire pour les amener à l'étape de séparation du méthane.

9. Procédé selon la revendication 6, comprenant en outre l'étape consistant à sous-refroidir le flux de condensat d'oléfines primaires pour l'étape de séparation de méthane.

10. Procédé selon la revendication 6, comprenant la séparation substantielle des composants C₄₊ à partir des effluents du four traités dans une colonne de distillation de dépropaniseur avant l'étape d'injection de liquide.

11. Procédé selon la revendication 2, comprenant la séparation substantielle des composants de C₃₊ du flux d'effluents de four traités dans une colonne de distillation de dééthaniseur avant l'étape d'injection de liquide.

12. Procédé selon la revendication 10, comprenant les étapes consistant à :
réaliser le strippage du flux de condensat d'oléfines primaires dans une colonne de préstrippage pour séparer les composants légers de celui-ci et générer un flux de condensat enrichi et un flux de vapeur pauvre secondaire ;
amener la vapeur pauvre secondaire vers l'étape de séparation de méthane ; et
amener le flux de condensat enrichi vers une colonne de distillation de dééthaniseur.

13. Installation de traitement d'oléfines, comprenant :
une unité de four (12) pour craquer des hydrocarbures et produire un flux d'effluents comprenant de l'hydrogène et des oléfines ;
une conduite (14) pour injecter un flux d'hydrocarbures liquides dans le flux d'effluents (26), produisant ainsi un flux conditionné (32) ;
une série de condensateurs et de séparateurs liquide-vapeur (27, 34) en cascade pour condenser et récupérer les oléfines du flux conditionné (32), et générer un flux de vapeur pauvre en oléfines réfrigéré ;
un séparateur de méthane (44) pour récupérer un flux de méthane issu des oléfines condensées ; et
un système de réfrigération pour fournir le réfrigérant primaire à un ou plusieurs des condensateurs en cascade comprenant une unité pour traiter le flux d'effluents du four en amont de la conduite d'injection de liquide, comprenant un compresseur et un séchoir en série, comprenant en outre :
une série d'échangeurs croisés en cascade (104, 124, 144) pour condenser partiellement les oléfines à partir d'une partie du flux d'effluents de four par échange thermique par rapport à des flux de vapeur pauvre en oléfines et de méthane récupéré réfrigérés ;
un dilatateur (172) pour dilater et refroidir encore les flux de vapeur pauvre en oléfines et de méthane récupéré ; et
des conduites (174, 176) pour diriger les flux de vapeur pauvre en oléfines et de méthane récupéré réfrigérés en tant que moyens d'échange thermique vers les échangeurs croisés (104, 124, 144).

14. Installation de traitement selon la revendication 13, comprenant un condensateur primaire associé opérationnellement à un séparateur de liquide-vapeur primaire pour condenser partiellement les oléfines issues du flux conditionné pour générer un flux de vapeur pauvre primaire pour l'amener aux condensateurs et séparateurs en cascade et un flux de condensat d'oléfines primaire.

15. Installation de traitement selon la revendication 14, dans laquelle le séparateur de méthane (44) comprend une unité de distillation de déméthaniseur.

16. Installation de traitement selon la revendication 14, comprenant une unité de distillation de dépropaniseur (59) pour séparer essentiellement les composants de C₄ et plus lourds de l'effluent de four traité avant injection du flux de conditionnement d'hydrocarbure liquide.

17. Installation de traitement selon la revendication 16, comprenant :
un pré-strippeur pour réaliser le strippage du flux de condensat d'oléfmes primaire pour séparer essentiellement les composants finaux légers de celui-ci et produire un flux de liquide enrichi et un flux de vapeur pauvre secondaire ;
une conduite pour amener le flux de vapeur pauvre secondaire vers l'unité de séparation de méthane ; et
une conduite pour amener le flux de liquide enrichi vers une colonne de distillation de dééthaniseur.
